# EUROPEAN PATENT APPLICATION

(11) **EP 4 567 124 A1**
(43) Date of publication of application: **11.06.2025**
(21) Application number: 23215043.3
(22) Date of filing: 07.12.2023
(51) Int. Cl.: C12P 17/04, C12N 9/16, C12N 9/88, C07D 307/50

(54) **BIOCATALYTIC PROCESS FOR THE PRODUCTION OF 4-HYDROXYMETHYLFURFURAL**

(71) Applicant: Cascat GmbH, 94315 Straubing (DE)
(72) Inventor: PICK, André, 36179 Bebra-Breitenbach (DE)
(74) Representative: Aera A/S

(57) **Abstract**

The present invention relates to a process for producing 4-hydroxymethylfurfural (HMF) comprising the steps of: converting 4-hydroxymethylfurfural-phosphate to 4-hydroxmethylfurfural using a phosphatase; and a process for producing 4-HMF from a feedstock comprising a carbon source, comprising the steps of converting 4-hydroxymethylfurfural-phosphate to 4-hydroxmethylfurfural using a phosphatase and an enzyme cascade comprising i.a. synthases to convert fructose-6-phosphate to glyceraldehyde-3-phosphate; wherein the carbon source is preferably selected from the group consisting of starch, dextrines, cellulose, sucrose and/or combinations thereof.

## Description

The present invention relates to a process for producing 4-hydroxymethylfurfural and a process for producing 4-HMF from a feedstock comprising a carbon source. Besides, the present invention relates to a phosphatase and the use of a phosphatase in the production of 4-hydroxmethylfurfural.

### BACKGROUND

5-Hydroxymethylfurfural represents an interesting precursor molecule for the generation of building blocks, like for example 2,5-Furandicarboxylic acid (2,5-FDCA), used for polymer synthesis. Routes for the generation of 5-Hydroxymethylfurfural use monosaccharides as substrate base, mainly fructose. The chemical conversion of fructose represents a dehydration process. 2,5-Furandicarboxylic acid (2,5-FDCA) has gained much attention due to its potential of substituting terephthalic acid in the synthesis of polyesters, specially polyethylene terephthalate (PET) (Sousa, Andreia F., et al. "Biobased polyesters and other polymers from 2,5-furandicarboxylic acid: a tribute to furan excellency." Polymer chemistry 6.33 (2015): 5961-5983). Substituting terephthalic acid to its furan analogue 2,5-FDCA in PET can lead to 2,5- furandicarboxylate (2,5-PEF) and this polymer has several advantages when compared to PET. In one aspect, 2,5-PEF has better thermal, barrier and mechanical properties when compared to its counterpart (PEP Report 294). Furthermore, as it is known that ethylene glycol could be produced from renewable resources, then 2,5-PEF could be 100% renewable as opposed to the semi renewable PET.

Despite all the aforementioned advantages of 2,5-FDCA in comparison to terephthalic acid, 2,5-FDCA production cost is still a current limitation in expanding the monomer usage. Existing technologies are not cost-competitive when compared to terephthalic acid. One of the possible reasons for this is related to the several sequential industrial steps required. One issue that could help reduce 2,5-FDCA production costs is finding a direct fermentation route from sugar to the desired molecule, but such a route has never been reported.

Up till now there exists no biocatalytic route for the generation of 5-hydroxymethylfurfural. In contrast, 4-hydroxymethylfurfural is found naturally in some microorganisms as a component of a more complex molecule called methanofuran. The biocatalytic synthesis leads to 4-(hydroxymethyl)-2-furancarboxaldehyde-phosphate which is converted by several additional steps into methanofuran. In the natural pathway 4-hydroxymethylfurfural does not occur in the microorganism only derivatives of this molecule.

Previous synthesis of 2,4-substituted furans, including 2,4-FDCA, required multiple synthetic steps and therefore 2,4-FDCA-derived polymers are cost-prohibitive by currently available methodologies and industrial techniques (Production of 4-Hydroxymethylfurfural from Derivatives of Biomass-Derived Glycerol for Chemicals and Polymers. ACS Sustainable Chem. Eng. 4(3):1707-1714).

As 4-HMF can be used as precursor for 2,4-bis(hydroxymethyl)furane, 2,4-furandicarboxylic acid, furan-2,4-dicarbaldehyde, 4-(hydroxymethyl)furoic acid, 2-formylfuran-4-carboxylate, 4-formylfuran-2-carboxylate and is a building block for polymers and plasticizer agents it would be of interest to have a production method leading to 4-HMF.

Hence, there is a need for a process for producing 4-HMF which allows the conversion of 4-hydroxymethylfurfural-phosphate to 4-hydroxmethylfurfural without a plurality of reaction steps and which could be implemented into a reaction cascade.

It is the object of the present invention to provide an efficient enzymatic conversion of 4-hydroxymethylfurfural-phosphate to 4-hydroxmethylfurfural, which can also be used in a reaction cascade. Minimizing the amounts of by-products and, if possible, allow recycling of reaction products in the cascade is also an object. Besides, a process being suitable for commercial and large-scale applications is also desirable.

The object is solved by a process for producing 4-hydroxymethylfurfural (HMF) comprising the step of converting 4-hydroxymethylfurfural-phosphate to 4-hydroxmethylfurfural using a phosphatase, a process for producing 4-HMF from a feedstock comprising a carbon source, a phosphatase and the use of a phosphatase in the production of 4-hydroxmethylfurfural.

In a first aspect the present invention relates to a process for producing 4-hydroxymethylfurfural (HMF) comprising, preferably consisting of, the step of:
- converting 4-hydroxymethylfurfural-phosphate to 4-hydroxmethylfurfural using a phosphatase.

Further embodiments are described in the dependent claims as well as in the description.

The present invention provides a biocatalytic process for the production of 4-HMF. The inventive process comprises, preferably consists of, the step of converting 4-hydroxymethylfurfural-phosphate to 4-hydroxmethylfurfural using a phosphatase.

Under phosphatase any enzyme falling within the classification EC 3.1.3. is to be understood.

Under transferase any enzyme falling within the classification EC 2.4. is to be understood. Under phosphorylase any enzyme falling within the classification EC 2.4.1. is to be understood. Under mutase any enzyme falling within the classification EC 5.4.2. is to be understood. Under isomerase any enzyme falling within the classification EC 5.3.1. is to be understood. Under hydrolase any enzyme falling within the classification EC 3.2. is to be understood. Under synthase any enzyme falling within the classification EC 4.2.3. is to be understood. Preferably the synthase is an enzyme falling within the classification EC 4.2.3.153.

4-HMF is a useful substrate as it can be converted in further compounds such as 2,4-Bis(hydroxymethyl)furane, 2,4-furandicarboxylic acid, furan-2,4-dicarbaldehyde, 4-(hydroxymethyl)furoic acid, 2-formylfuran-4-carboxylate, or 4-formylfuran-2-carboxylate.

So in one embodiment the 4-HMF is further converted into a compound selected from the group consisting of 2,4-Bis(hydroxymethyl)furane, 2,4-furandicarboxylic acid, furan-2,4-dicarbaldehyde, 4-(hydroxymethyl)furoic acid, 2-formylfuran-4-carboxylate, 4-formylfuran-2-carboxylate or combinations thereof.

The inventive process has the advantage that a phosphatase can produce 4-hydroxmethylfurfural from 4-hydroxymethylfurfural-phosphate in one step. One of the advantages is that no products apart of 4-HMF and phosphate are formed. This reduces the production of unwanted side-products which makes purification straight forward and cheaper. Apart of that the addition of a phosphatase reduces the costs as well. Besides, the phosphate can be recycled in the process.

In addition, in an embodiment the conversion of 4-hydroxymethylfurfural-phosphate to 4-hydroxmethylfurfural using the phosphatase is an irreversible reaction.

The enzymes useful in the present invention are either wild-type enzymes or have undergone certain genetic modifications that improve the reactivity towards 4-hydroxymethylfurfural-phosphate as substrate. The enzymes with desirable properties, such as ability to use phosphorylated substrates, which are derived from the wild type enzymes through recombinant technology are referred to as mutants, mutant derivatives or mutants thereof from the corresponding wild type enzymes.

The classification or discussion of a material in any section of this specification as having a particular utility (e.g. catalyst) is for convenience.

Sugars or carbohydrates as used in this invention refer to a biological molecule consisting of carbon, hydrogen and oxygen atoms, usually with hydrogen: oxygen atom ratio of 2 : 1; in other words with the empirical formula Cₘ(H₂O)ₙ (where m could be different from n).

When referring to a substance occurring as enantiomers such as e.g. D-glyceraldehyde (DGA) and L-glyceraldehyde (LGA) or e.g. L-fructose and D-fructose both isomers of this substance are covered when e.g. the term glyceraldehyde or fructose is used as long as no specific isomer is mentioned.

The citation of references herein does not constitute an admission that such references are prior art or relevant to the patentability of the invention disclosed herein. Any discussion of the contents of the references cited is intended to provide only a general summary of the claims made by the authors of the references and does not constitute an admission as to the correctness of the contents of such references.

While the description and specific examples provide embodiments of the invention, they are for illustrative purposes only and are not intended to limit the scope of the invention. Furthermore, mention of several embodiments having the features mentioned is not intended to exclude other embodiments having additional features or other embodiments having other combinations of the features mentioned. Specific examples are provided to illustrate how the substances and processes of the present invention may be made and used and, unless otherwise expressly stated, are not to be construed as representations that particular embodiments of the present invention have or have not been made or tested.

As used herein, the words preferred and preferably refer to embodiments of the invention that provide certain advantages under certain circumstances. However, other embodiments may also be preferred under the same or different circumstances. Moreover, mention of one or more preferred embodiments does not imply that other embodiments are not useful and is not intended to exclude other embodiments from the scope of the invention.

The 4-hydroxymethylfurfural-phosphate applied in the present invention can be derived from various sources.

Sources are e.g. biomass such as complex carbohydrates. The 4-hydroxymethylfurfural-phosphate used in this invention can be derived from biological sources such as carbohydrates.

The novel direct biocatalytic route allows the 4-HMF production providing glyceraldehyde-3-phosphate as an intermediate from carbon feedstock such as starch, glycogen, maltodextrin, cellulose, hemicellulose, sucrose with commercial applicability on an industrial scale.

The processes to obtain 4-hydroxymethylfurfural-phosphate can be chemical and/or enzymatical. It is preferred according to the present invention that those processes are enzymatical processes.

Using 4-hydroxymethylfurfural-phosphate as substrate biocatalytic reactions are employed to form carbohydrates and other chemicals.

Surprisingly, a process for producing 4-HMF comprising the step(s) of converting 4-hydroxymethylfurfural-phosphate to 4-hydroxmethylfurfural using a phosphatase has been found. This process yields 4-HMF in high yield and tolerates the presence of other substances as well. Apart of that the process works under mild conditions, that is, without high pressures and temperatures and better tolerates the presence of in-process contaminants. Additionally, the use of co-factors like NAD⁺/NADH, NADP⁺/NADPH, FAD, FMN or ATP, is not required. As a result, a higher proportion based on the substrates can be achieved.

According to an embodiment the phosphatase is obtained from an organism, preferably a microorganism and more preferably from a microorganism selected from the group consisting of *Escherichia coli* and/or *Blautia obeum.*

According to an embodiment the phosphatase has an activity towards other compounds present in the process less than or equal to 10% compared to the activity towards 4-hydroxymethylfurfural-phosphate. From this follows that the phosphatase has a high specificity towards 4-hyd roxymethylfu rfu ral-phosphate.

The comparison in activity is calculated by using the amount of phosphate produced by an enzyme (cf. e.g. Table 1) reduced by the blank value without the presence of the enzyme (cf. e.g. Table 1) and these are set in relation.

In an embodiment the phosphatase has an activity towards glucose-1-phosphate, glucose-6-phosphate, fructose-6-phosphate and glyceraldehye-3-phosphate, which might be intermediates in a reaction cascade, being less than or equal to 10% compared to the activity towards 4-hydroxymethylfurfural-phosphate.

In another embodiment the phosphatase has a high specificity towards 4-hydroxymethylfurfural-phosphate.

In a further embodiment the phosphate created in the conversion from 4-hydroxymethylfurfural-phosphate to 4-hydroxmethylfurfural is recycled.

In another embodiment in the conversion from 4-hydroxymethylfurfural-phosphate to 4-hydroxmethylfurfural no co-factor, preferably no NAD⁺/NADH, NADP⁺/NADPH, FAD, FMN or ATP, is needed.

In a further embodiment the phosphate created in the production of 4-hydroxmethylfurfural is recycled in the production process.

This can be for example one phosphate which is generated when 4-hydroxymethylfurfural-phosphate is prepared from glyceraldehyde-3-phosphate and another phosphate which is generated by the dephosphorylation of 4-hydroxymethylfurfural-phosphate to obtain 4-HMF. These phosphates can e.g. be used to generate glucose-1-phosphate.

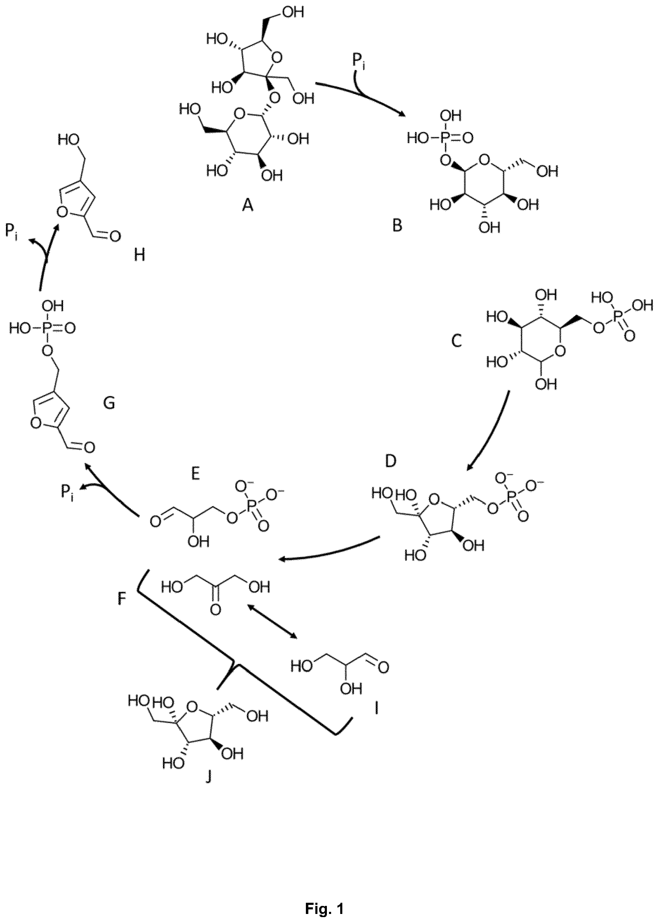

A potential reaction cascade in which the inventive process for producing 4-hydroxymethylfurfural (HMF) comprising the steps of converting 4-hydroxymethylfurfural-phosphate to 4-hydroxmethylfurfural using a phosphatase, is part of is shown in Fig. 1. Processes like the aforementioned are driven by the incorporation of phosphate in one of its first biocatalytic steps and its release in the later or last steps. The incorporation takes place with the generation of glucose-1-phosphate and the release is catalyzed with the generation of 4-hydroxymethylfurfural-phosphate from two glyceraldehyde-3-phosphate molecules as well as from the cleavage of 4-hydroxymethylfurfural-phosphate into 4-hydroxymethylfurfural and phosphate.

According to one embodiment 4-hydroxymethylfurfural-phosphate is prepared from glyceraldehyde-3-phosphate using at least one enzyme.

In a further embodiment 4-hydroxymethylfurfural-phosphate is prepared from glyceraldehyde-3-phosphate using at least one enzyme and wherein the phosphate created in the conversion is recycled.

In one embodiment the at least one enzyme is a synthase.

In another embodiment the at least one enzyme is a synthase and the synthase is obtained from an organism, preferably a microorganism and more preferably from a microorganism selected from the group consisting of *Methanocaldococcus jannaschii, Methanococcus maripaludis, Methanothermococcus okinawensis, Methanobacterium paludism, Methanobrevibacter wolinii, Methylomonas methanica, Methanosarcina mazei, and Methanothermococcus thermolithotrophicus.*

In an embodiment the glyceraldehyde-3-phosphate is prepared from fructose-6-phosphate using at least one enzyme, wherein this reaction also yields dihydroxyacetone (DHA) in a non-phosphorylated form. Preferably the DHA is isomerized to glyceraldehyde using an isomerase; wherein more preferably the isomerase is an isomerase belonging to the EC5.3.1 class, even more preferably the isomerase is an isomerase belonging to the EC5.3.1.22 class or EC5.3.1.35 class. Also preferred the isomerase is a hydroxypyruvate isomerase or a 2-dehydrotetronate isomerase.

It is further preferred that the DHA is further converted into fructose, preferably the mixture of DHA and glyceraldehyde is converted into fructose.

In one embodiment the synthase comprises an amino acid sequence that is at least 80 %, preferably at least 90 %, more preferably at least 98 %, even more preferably at least 98.5%, and even more preferably at least 99 % identical with the sequence according to SEQ ID NO: 23.

In another embodiment the synthase has an amino acid sequence according to SEQ ID NO: 15, SEQ ID NO: 17, SEQ ID NO: 19, SEQ ID NO: 21, SEQ ID NO: 25, SEQ ID NO: 27, or SEQ ID NO: 29.

In another embodiment the process is cell-free. An easy conversion of 4-hydroxymethylfurfural-phosphate to 4-hydroxmethylfurfural allows the implementation of a cell-free process. No loss of substrate within the metabolism and a straightforward conversion that tolerates higher product concentrations are benefits of a cell-free process.

In an embodiment the process is part of a multi-enzymatic cascade.

In an embodiment the enzymatic conversion is a one-pot reaction.

Preferably the inventive process is carried out at suitable temperatures, e.g. may depend on the enzymes used. Suitable temperatures include 10 - 100 °C, preferably 10 - 90 °C, more preferably 20 - 90 °C, and even more preferably 20 - 80 °C.

Preferably the temperature in the conversion of 4-hydroxymethylfurfural-phosphate to 4-hydroxmethylfurfural is between 10 - 100 °C, more preferably between 20 - 90 °C and even more preferably between 20 - 80 °C.

Preferably the temperature during all conversion steps is between 10 - 100 °C, more preferably between 20 - 90 °C and even more preferably between 20 - 80 °C.

Further preferred the pH during the conversion of 4-hydroxymethylfurfural-phosphate to 4-hydroxmethylfurfural is between 3 - 12 and preferably between 4 - 10.

Further preferred the pH during all conversion steps is between 3 - 12 and preferably between 4 - 10.

The inventive process can be used with a limited set of co-factors. In particular, the inventive process does not lead to net ATP production and does not need ATP. Apart of that it is preferred that the inventive process uses only enzymes that withstand the inactivating presence of the produced chemicals.

In one embodiment the phosphatase comprises an amino acid sequence that is at least 95 %, preferably at least 98 %, more preferably at least 98.5 % and even more preferably at least 99 % identical with the sequence according to SEQ ID NO: 51, preferably the phosphatase is derived from *E. coli.*

In another embodiment the phosphatase comprises an amino acid sequence that is at least 80 %, preferably at least 90 %, and more preferably at least 98 % and even more preferably at least 99 % identical with the sequence according to SEQ ID NO: 35, preferably the phosphatase is derived from a *Blautia obeum.*

According to another embodiment the phosphatase has an amino acid sequence according to SEQ ID NO: 35, SEQ ID NO: 37, SEQ ID NO: 47, SEQ ID NO: 49, SEQ ID NO: 51, or SEQ ID NO: 53.

In another aspect the present invention relates to a process for producing 4-HMF from a feedstock comprising a carbon source, comprising the steps of
- converting 4-hydroxymethylfurfural-phosphate to 4-hydroxmethylfurfural using a phosphatase;
wherein the carbon source is preferably selected from the group consisting of starch, dextrines, cellulose, sucrose and/or combinations thereof.

It should be understood that any feature and/or aspect discussed above in connection with the process for producing 4-hydroxymethylfurfural (HMF) according to the invention apply by analogy to the process for producing 4-HMF from a feedstock comprising a carbon source described herein.

In this process saccharide phosphates are intermediary produced.

According to an embodiment saccharide phosphates are intermediary produced and the enzymatic conversion is a one-pot reaction.

In one embodiment the following intermediates are generated: glucose-1-phosphate, glucose-6-phosphate, fructose-6-phosphate and glyceraldehye-3-phosphate.

According to an embodiment at least one transferase, preferably a glycosyltransferase, more preferably a glucanotransferase, even more preferably an alpha-glucanotransferase is used; and/or at least one phosphorylase, preferably a glucanphosphorylase is used; and/or at least one mutase, preferably a phosphoglucomutase is used; and/or at least one isomerase, preferably a phosphoglucoisomerase is used; and/or at least one hydrolase, preferably a glucanohydrolase, more preferably pullulanase is used.

In an embodiment of the process for producing 4-HMF from a feedstock comprising a carbon source at least five enzymes, preferably at least six enzymes, are used.

In the inventive process according to one embodiment a reaction sequence is applied, enabling the cleavage of individual D-glucose molecules in the form of D-glucose-1-phosphate. Subsequently, these released D-glucose-1-phosphate molecules are converted via further saccharide phosphate intermediates into D-fructose-6-phosphate. D-fructose-6-phosphate is subsequently split, and D-glyceraldehyde and dihydroxyacetone are obtained. Reaction steps during this conversion sequence comprise the following steps:
converting a saccharide molecule derived from a feedstock comprising a carbon source enzymatically into D-glucose-1-phosphate, and
converting D-glucose-1-phosphate enzymatically to D-glucose-6-phosphate, and
converting D-glucose-6-phosphate enzymatically to D-fructose 6-phosphate, and
converting D-fructose 6-phosphate to D-glyceraldehyde-3-phosphate and dihydroxyacetone.

In an embodiment the carbon source is selected from the group consisting of starch, dextrines, cellulose, sucrose and/or combinations thereof.

In another embodiment the process for producing 4-HMF from a feedstock comprising a carbon source is a multi-enzyme reaction cascade.

According to an embodiment the phosphate created in the production of 4-hydroxmethylfurfural is recycled in the production process.

In another embodiment the phosphate created in the conversion from 4-hydroxymethylfurfural-phosphate to 4-hydroxmethylfurfural is recycled in the process.

In one embodiment no co-factor, preferably no NAD⁺/NADH, NADP⁺/NADPH, FAD, FMN or ATP, is needed.

According to one embodiment the phosphatase has an activity towards other compounds present in the process being less than or equal to 10% compared to the activity towards 4-hydroxymethylfurfural-phosphate, preferably the phosphatase has an activity towards glucose-1-phosphate, glucose-6-phosphate, fructose-6-phosphate and glyceraldehye-3-phosphate being less than or equal to 10% compared to the activity towards 4-hydroxymethylfurfural-phosphate.

In another aspect the invention relates to a phosphatase for converting 4-hydroxymethylfurfural-phosphate to 4-hydroxmethylfurfural.

It should be understood that any feature and/or aspect discussed above in connection with the processes according to the invention described above apply by analogy to the phosphatase described herein.

The phosphatase for converting 4-hydroxymethylfurfural-phosphate to 4-hydroxmethylfurfural comprises an amino acid sequence that is at least 95 %, preferably at least 98 %, more preferably at least 98.5 % and even more preferably at least 99 % identical with the sequence according to SEQ ID NO: 51, preferably the phosphatase is derived from *E. coli;* or
the phosphatase for converting 4-hydroxymethylfurfural-phosphate to 4-hydroxmethylfurfural comprises an amino acid sequence that is at least 80 %, preferably at least 90 %, and more preferably at least 98 % and even more preferably at least 99 % identical with the sequence according to SEQ ID NO: 35, preferably the phosphatase is derived from a *Blautia obeum.*

In another embodiment the phosphatase has an amino acid sequence according to SEQ ID NO: 35, SEQ ID NO: 37, SEQ ID NO: 47, SEQ ID NO: 49, SEQ ID NO: 51, or SEQ ID NO: 53.

In another aspect the present invention relates to the use of a phosphatase in the production of 4-hydroxmethylfurfural.

It should be understood that any feature and/or aspect discussed above in connection with the processes and the phosphatase according to the invention described above apply by analogy to the use of a phosphatase in the production of 4-hydroxmethylfurfural described herein.

### Brief description of the figure

- **Fig. 1**: shows the process of the conversion of sucrose to 4-HMF with the further production of fructose (A = sucrose, B = glucose-1-phosphate, C = glucose-6-phosphate, D = fructose-6-phosphate, E = glyceraldehyde-3-phosphate, F = dihydroxyacetone, G = 4-HMF-phosphate, H = 4-HMF, I = glyceraldehyde, J = fructose; Pᵢ = phosphate).

In the following the abbreviations and the enzymes used in the figure and/or tables are explained:
*Bf*PulA - *Bacillus flavocaldarius* KP1228 Pullulanase (SEQ ID No. 1)
*Mr*Agt - *Meiothermus ruber* alpha-Glucanotransferase (SEQ ID No. 3)
*Aa*Agp - *Aquifex aeolicus* alpha-Glucan Phosphorylase (SEQ ID No. 5)
*Mr*Agp - *Meiothermus ruber* alpha-Glucan Phosphorylase (SEQ ID No. 7)
*Mr*Pgm- *Meiothermus ruber* Phosphoglucomutase (SEQ ID No. 9)
*Mr*Pgi - *Meiothermus ruber* Phosphoglucoisomerase (SEQ ID No. 11)
*Ec*FsaA - *Escherichia coli* Fructose-6-phosphate aldolase (SEQ ID No. 13)
*Ec*FsaA A129S - *Escherichia coli* Fructose-6-phosphate aldolase variant A129S (SEQ ID No. 41)*Mj*MfnB - *Methanocaldococcus jannaschii* 4-(hydroxymethyl)-2-Furancarboxaldehyde phosphate synthase (SEQ ID No. 15)
*Mm*MfnB - *Methanococcus maripaludis* 4-(hydroxymethyl)-2-Furancarboxaldehyde phosphate synthase (SEQ ID No. 17)
*Mo*MfnB - *Methanothermococcus okinawensis* 4-(hydroxymethyl)-2-Furancarboxaldehyde phosphate synthase (SEQ ID No. 19)
*Mp*MfnB - *Methanobacterium paludis* 4-(hydroxymethyl)-2-Furancarboxaldehyde phosphate synthase (SEQ ID No. 21)
*Mw*MfnB - *Methanobrevibacter wolinii* 4-(hydroxymethyl)-2-Furancarboxaldehyde phosphate synthase (SEQ ID No. 23)
*Mm*eMfnB - *Methylomonas methanica* 4-(hydroxymethyl)-2-Furancarboxaldehyde phosphate synthase (SEQ ID No. 25)
*Mm*aMfnB - *Methanosarcina mazei* 4-(hydroxymethyl)-2-Furancarboxaldehyde phosphate synthase (SEQ ID No. 27)
*Mt*MfnB - *Methanothermococcus thermolithotrophicus* 4-(hydroxymethyl)-2-Furancarboxaldehyde phosphate synthase (SEQ ID No. 29)
*Ec*YidA - *Escherichia coli* Phosphatase YidA (SEQ ID No. 55)
*Ec*YihX - *Escherichia coli* Phosphatase YihX (SEQ ID No. 57)
*Ec*AppA - *Escherichia coli* Phosphoanhydride phosphorylase (SEQ ID No. 59)
*Ec*HxpA - *Escherichia coli* Phosphatase HxpA (SEQ ID No. 61)
*Ec*GlpX - *Escherichia coli* Phosphatase GlpX (SEQ ID No. 63)
*Ec*Ffp - *Escherichia coli* Fructose-1-phosphate phosphatase (SEQ ID No. 31)
*Ec*Ffp N68D - *Escherichia coli* Fructose-1-phosphate phosphatase (SEQ ID No. 47)
*Ec*Ffp N68E - *Escherichia coli* Fructose-1-phosphate phosphatase (SEQ ID No. 49)
*Ec*Ffp S178N - *Escherichia coli* Fructose-1-phosphate phosphatase (SEQ ID No. 51)
*Ec*Ffp S178M - *Escherichia coli* Fructose-1-phosphate phosphatase (SEQ ID No. 53)
*El*Ffp - *Eubacterium limosum* Fructose-1-phosphate phosphatase (SEQ ID No. 33)
*Bo*Ffp - *Blautia obeum* Fructose-1-phosphate phosphatase (SEQ ID No. 35)
*Wc*Ffp - *Weissella cibaria* Fructose-1-phosphate phosphatase (SEQ ID No. 37)
*Ba*Scp - *Bifidobacterium adolescentis* Sucrose phosphorylase (SEQ ID No. 65)
*Ct*GapN - *Clostridium thermocellum* glyceraldehyde-3-phosphate dehydrogenase (SEQ ID No. 39)
*Ct*CdeP - *Clostridium thermocellum* cellodextrine phosphorylase (SEQ ID No. 67)
*Ct*CelP - *Clostridium thermocellum* cellobiose phosphorylase (SEQ ID No. 69)
*Ec*Otnl - Escherichia coli 2-dehydrotetronate isomerase (SEQ ID No. 43)
*Pw*Hyi - Polynucleobacter wuianus hydroxypyruvate isomerase (SEQ ID No. 45)

While the invention has been described with reference to certain embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiments, but that the invention will include all embodiments falling within the scope of the appended claims.

### EXPERIMENTAL SECTION

### 1. Materials

All chemicals were of analytical grade or higher quality and purchased from Sigma-Aldrich, Biosynth or VWR and used without further purification. Substrates used in the processes starch, maltodextrin, cellulose, cellodextrins and sucrose were purchased from Sigma-Aldrich, Biosynth or VWR. The following strains were used during this work; *E. coli* XL1 Blue, *E. coli* BL21(DE3), every construct was successful expressed using autoinduction media at 37°C.

### 2. Methods

### Analytics

Analytics for the following components glucose, fructose, glucose-1-phosphate, glucose-6-phosphate, fructose-6-phosphate were performed using a HPLC Dionex Ultimate 3000 system equipped with autosampler (WPS 3000TRS), a column compartment (TCC3000RS) and a light scattering detector. The YMC Triart Diol Hilic column (100 * 2 mm, 1.9 µm, 12 nm) at 7 °C was used for separation by gradient elution (15-85%) with 0.1% formic acid pH 4.5, and 0.1% formic acid in ACN as the mobile phase at 0.45 ml min⁻¹. Samples were diluted in water/acetonitrile with the ratio of 3:7. Data were analyzed using Dionex Chromeleon software.

Glucose is detected using an assay based on glucose oxidase. 50 µl of the sample or diluted sample is mixed with 50 µl of master mix (0.75 mM ABTS, 2 U/ml glucose oxidase, 0.1 U/ml Peroxidase, 20 mM KPi pH 6.0) and incubated for 30 min at 30°C and then measured at 418 nm using a spectrophotometer (Multiskan GO, Thermo Fisher).

### Protein expression

Optimized protein expression is exemplarily described for one enzyme and was performed for all proteins by the same procedure.

*E. coli* BL21 (DE3) containing the plasmid of interest (pET28 derivative with one gene encoding one of the described enzymes for the process) was grown in 250 ml autoinduction media (Studier, F. William. "Protein production by auto-induction in high-density shaking cultures." Protein expression and purification 41.1 (2005): 207-234.). The preculture was incubated in 4 ml of LB medium with 100 µg/ml kanamycin at 37 °C overnight on a rotary shaker (180 rpm). Expression culture was inoculated with a 1:100 dilution of overnight culture. Incubation was performed for 24 h at 37 °C. Cells were harvested by centrifugation and resuspended in 50 mM TRIS-HCl (pH 8.0). Crude extracts were prepared by use of a Basic-Z Cell Disrupter (IUL Constant Systems) or an ultrasonic device and subsequent addition of MgCl₂ to a final concentration of 2.5 mM in combination with DNasel (1 µg/ml) and a following incubation for 20 min at room temperature (25°C) to degrade DNA. Afterwards all crude extracts were heat treated for 30 min at 70 °C. The insoluble fraction of the lysate was removed by centrifugation (20,000 rpm for 40 min at 4 °C). The supernatant was filtered through a 0.45 µm syringe filter and used as purified enzyme preparation. Aliquots of each purification were subjected to 12 % SDS-Page described by Laemmli Ulrich K. "Cleavage of structural proteins during the assembly of the head of bacteriophage T4." nature 227.5259 (1970): 680.).

The enzymes correspond to the following sequences SEQ ID No. 1, SEQ ID No. 3, SEQ ID No. 5, SEQ ID No. 7, SEQ ID No. 9, SEQ ID No. 11, SEQ ID NO: 13, SEQ ID NO: 15, SEQ ID NO: 17 , SEQ ID NO: 19, SEQ ID NO: 21, SEQ ID NO: 23, SEQ ID NO: 25, SEQ ID NO: 27 , SEQ ID NO: 29, SEQ ID NO: 31, SEQ ID NO: 33, SEQ ID NO: 35, SEQ ID NO: 37 , SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53.

### Activity testing of enzymes

Pullulanase of *Bacillus flavocaldarius* (SEQ ID No. 1) was tested for activity using pullulan as substrate and 3,5-dinitrosalicylic acid (DNS) for detection. Pullulanase was incubated with 1 % (w/v) pullulan in 50 mM potassium phosphate buffer pH 7.0 and 5 mM MgCl₂ at 65 °C. At different time points an aliquot of 24 µl was transferred into 96 µl of DNS reagent (10 g 3,5-dinitrosalicylic acid, 300 g sodium potassium tartrate, 16 g sodium hydroxide for 1 liter). Heated up to 95 °C for 5 minutes and afterwards were 100 µl transferred into a flat bottom microtitre plate and measured at 540 nm in a spectrophotometer (Multiskan GO, Thermo Fisher).

α-Glucanotransferase of *Meiothermus ruber* (SEQ ID No. 3) was tested for activity using maltotriose as substrate and the ability to build up larger oligosaccharides. Detection of activity was done using TLC (Isopropanol:Ethylacetate:Water (3:1:1)) and thymol spray reagent (0.5 g thymol 95 ml ethanol 5 ml sulfuric acid 97%). α-Glucanotransferase of *Meiothermus ruber* was incubated with 50 mM maltotriose in 50 mM potassium phosphate buffer pH 7.0 and 5 mM MgCl₂ at 20 -50 °C.

α-Glucan phosphorylase of *Aquifex aeolicus* (SEQ ID No. 5) or *Meiothermus ruber* (SEQ ID No. 7) was tested for activity using a combination of enzymes to detect the product α-D-glucose-1-phosphate. α-D-Glucose-1-phosphate was converted to α-D-glucose-6-phosphate which was subsequently oxidized by α-D-glucose-6-phosphate dehydrogenase to α-D-gluconate-6-phosphate and NADH was detected. α-Glucan phosphorylase was incubated with 1 % (w/v) soluble starch in 50 mM potassium phosphate buffer pH 7.0 and 5 mM MgCl₂ and 0.1 mM Pyridoxal-5-phosphate at 20 - 65 °C. At different time points an aliquot of 10 µl was transferred into 190 µl of detection solution containing phosphoglucomutase 0.1 U, α-D-glucose-6-phosphate dehydrogenase 0.1 U, 1 mM NAD⁺. The mixture was incubated for 30 min and the measured at 340nm in a spectrophotometer (Multiskan GO, Thermo Fisher).

Sucrose Phosphorylase of *Bifidobacterium adolescentis* (SEQ ID No. 65) was tested for activity using sucrose as substrate and 3,5-dinitrosalicylic acid for detection. In an assay using 3,5-dinitrosalicylic acid for detection only the product fructose produces a signal. Sucrose Phosphorylase was incubated with 100 mM sucrose in 100 mM potassium phosphate buffer pH 7.0 and 5 mM MgCl₂ at 20 - 50 °C. At different time points an aliquot of 24 µl was transferred into 96 µl of DNS reagent (10 g 3,5-dinitrosalicylic acid, 300 g sodium potassium tartrate, 16 g sodium hydroxide for 1 liter). Heated up to 95 °C for 5 min and afterwards were 100 µl transferred into a flat bottom microtitre plate and measured at 540 nm in a spectrophotometer (Multiskan GO, Thermo Fisher).

Phosphoglucomutase of *Meiothermus ruber* (SEQ ID No. 9) was tested for activity using α-D-glucose-1-phosphate as substrate and 3,5-dinitrosalicylic acid for detection. In an assay using 3,5-dinitrosalicylic acid for detection only the product α-D-glucose-6-phosphate produces a signal. Phosphoglucomutase was incubated with 50 mM α-D-glucose-1-phosphate in 50 mM potassium phosphate buffer pH 7.0 and 5 mM MgCl₂ at 20 - 50 °C. At different time points an aliquot of 24 µl was transferred into 96 µl of DNS reagent (10 g 3,5-dinitrosalicylic acid, 300 g sodium potassium tartrate, 16 g sodium hydroxide for 1 liter). Heated up to 95 °C for 5 min and afterwards were 100 µl transferred into a flat bottom microtitre plate and measured at 540 nm in a spectrophotometer (Multiskan GO, Thermo Fisher).

Phosphoglucoisomerase of *Meiothermus ruber* (SEQ ID No. 11) was tested for activity using fructose-6-phosphate as substrate and α-D-glucose-6-phosphate dehydrogenase for detection. The produced NADH was detected at 340 nm. Phosphoglucoisomerase was incubated with 50 mM fructose-6-phosphate in 50 mM potassium phosphate buffer pH 7.0 and 5 mM MgCl₂ at 20 - 50 °C. At different time points an aliquot of 10 µl was transferred into 190 µl of detection solution containing α-D-glucose-6-phosphate dehydrogenase 0.1 U, 1 mM NAD⁺. The mixture was incubated for 30 min and the measured at 340nm in a spectrophotometer (Multiskan GO, Thermo Fisher).

Fructose-6-phosphate aldolase of *Escherichia coli* (SEQ ID No. 13) or Fructose-6-phosphate aldolase A129S of *Escherichia coli* (SEQ ID No. 41) was tested for activity using Fructose-6-phosphate as substrate. Together with glyceraldehyde-3-phosphate dehydrogenase from *Clostridium thermocellum* (SEQ ID No. 39) the generation of glycerlaldehyde-3-phosphate was detected through the oxidation to glycerate-3-phosphate with the generation of NADPH from NADP⁺ in 50 mM potassium phosphate buffer pH 7.0 and 5 mM MgCl₂ at 20 -50 °C. Tests were performed in a flat bottom microtiter plate and measured at 340 nm in a spectrophotometer (Multiskan GO, Thermo Fisher).

Phosphatases of *Escherichia coli* (SEQ ID No. 31, SEQ ID No. 55, SEQ ID No. 57, SEQ ID No. 59, SEQ ID No. 61, SEQ ID No. 63) and variants of Ffp of *Escherichia coli* (SEQ ID No. 47, SEQ ID No. 49, SEQ ID No. 51, SEQ ID No. 53), *Eubacterium limosum* (SEQ ID No. 33), *Blautia obeum* (SEQ ID No. 35), *Weissella cibaria* (SEQ ID No. 37) was tested for activity using several substrates (glucose-1-phosphate, glucose-6-phosphate, fructose-6-phosphate, glyceraldehyde-3-phosphate) and a phosphate assay. The phosphate assay consists of three solutions, solution A (12 % (w/v) L-ascorbic acid in 1N HCl solution), solution B (2 % (w/v) Na₂MoO₄ x 2H₂O in ddH₂O), solution F (2 % (w/v) citric acid and 2 % (w/v) acetic acid in ddH₂O). Solution D is a 2:1 mixture of solution A and solution B and prepared freshly before the measurement. 75 µl of solution D is pipetted into the needed wells of a flat-bottom microtiter plate. In the next step 25 µl of the sample or a standard with defined concentration is added and incubated for 5 min at room temperature. In the last step the reaction is stopped by addition of 75 µl of solution F and an additional incubation step of 15 min at room temperature. The samples are measured at 655 nm in a spectrophotometer (Multiskan GO, Thermo Fisher). Phosphatase was incubated with 50 mM substrate in 50 mM TRIS-HCl buffer pH 7.0 and 5 mM MgCl₂ at 20 -50 °C. At different time points an aliquot of 25 µl was removed.

Escherichia coli 2-dehydrotetronate isomerase (SEQ ID No. 43) or Polynucleobacter wuianus hydroxypyruvate isomerase (SEQ ID No. 45) were tested for activity using dihydroxyacetone as substrate. Together with glyceraldehyde dehydrogenase variant from *Thermoplasma acidophilum* (Steffler, Fabian, Jan-Karl Guterl, and Volker Sieber. "Improvement of thermostable aldehyde dehydrogenase by directed evolution for application in synthetic cascade biomanufacturing." Enzyme and microbial technology 53.5 (2013): 307-314.) the generation of glycerate was detected through the generation of NADH from NAD⁺ in 50 mM potassium phosphate buffer pH 7.0 and 5 mM MgCl₂ at 25 -50 °C. Tests were performed in a flat bottom microtiter plate and measured at 340 nm in a spectrophotometer (Multiskan GO, Thermo Fisher).

All tested enzymes fulfill the requirements to catalyze one of the steps within the process and are stable under process conditions (20 - 50 °C, 50 mM potassium phosphate buffer pH 7.0, 5 mM MgCl₂ 0.005 mM PLP).

### 3. Substrate acceptance phosphatases

### Testing Escherichia coli phosphatases

For monitoring the different activities of the different phosphatases and the variants for four saccharide-phosphate intermediates occurring in the process a phosphate assay was performed.

The reaction mixture contained 50 mM HEPES buffer pH 7.0, 10 mM MgCl₂, 10 mM of G1P, G6P, F6P, Gly3P and one phosphatase (0.05 mg/ml) in a total volume of 0.1 ml. The mixture was incubated at 25 °C and in regular time intervals an aliquot was removed and tested for released phosphate. The phosphate assay is described above in further detail.

The results of the phosphate assay are shown in Table 1. The amount of release phosphate [mM] was detected and is listed in Table 1.

The enzymes correspond to the following sequences SEQ ID NO: 31, SEQ ID NO: 55, SEQ ID NO: 57, SEQ ID NO: 59, SEQ ID NO: 61.

**Table 1: Comparison of different Escherichia coli phosphatases for different substrates occurring in the 4-HMF production process**

| Phosphate [mM] | | | | | | | |
|---|---|---|---|---|---|---|---|
| | EcFfp | EcYidA | EcYihX | EcAppA | EcHxpA | EcGIpX | blank |
| F6P | 0.63 | 0.59 | 0.03 | 4.40 | 0.36 | 0.12 | 0.07 |
| G6P | 1.99 | 0.34 | 0.06 | 4.69 | 0.45 | 0.05 | 0,01 |
| G1P | 0.02 | 0.49 | 1.12 | 2.51 | 0.03 | 0.8 | 0.00 |
| Gly3P | 0.09 | 0.08 | 0.05 | 3.4 | 0.09 | 0.04 | 0.03 |
| 4-HMF-P | 0.85 | 0.32 | 0.10 | 3.72 | 0.07 | 0.09 | 0.07 |

### Testing Escherichia coli phosphatases and variants

For monitoring the different activities of the different phosphatases and the variants for four saccharide-phosphate intermediates occurring in the process a phosphate assay was performed.

The reaction mixture contained 50 mM HEPES buffer pH 7.0, 10 mM MgCl₂, 10 mM of G1P, G6P, F6P, Gly3P and one phosphatase (0.005 mg/ml) in a total volume of 0.1 ml. The mixture was incubated at 25 °C and in regular time intervals an aliquot was removed and tested for released phosphate. The phosphate assay is described above in further detail.

The results of the phosphate assay are shown in Table 2. The amount of release phosphate was detected.

The enzymes correspond to the following sequences SEQ ID NO: 31, SEQ ID NO: 47, SEQ ID NO: 49, SEQ ID NO: 51, SEQ ID NO: 53.

**Table 2: Comparison of Escherichia coli phosphatase Ffp and variants of this phosphatase for different substrates occurring in the 4-HMF production process**

| Phosphate [mM] | | | | | | |
|---|---|---|---|---|---|---|
| | EcFfp | EcFfpN68D | EcFfpN68E | EcFfpS178N | EcFfpS178M | blank |
| F6P | 0.17 | 0.15 | 0.12 | 0.09 | 0.16 | 0.07 |
| G6P | 1.03 | 0.45 | 0.05 | 0.03 | 0.02 | 0,01 |
| G1P | 0.02 | 0.01 | 0.01 | 0.02 | 0.01 | 0.01 |
| Gly3P | 0.03 | 0.03 | 0.04 | 0.02 | 0.03 | 0.01 |
| 4-HMF-P | 0.29 | 0.15 | 0.25 | 0.34 | 0.23 | 0.02 |

Identification of the positions N68 and S178 responsible for the acceptance of the substrate molecules allowed the improvement towards 4-HMF-P cleavage and reducing the activities towards the other phosphorylated intermediates almost completely.

### Testing phosphatases from different microorganisms

For monitoring the different activities of the different phosphatases and the variants for four saccharide-phosphate intermediates occurring in the process a phosphate assay was performed.

The reaction mixture contained 50 mM HEPES buffer pH 7.0, 10 mM MgCl₂, 10 mM of G1P, G6P, F6P, Gly3P and one phosphatase (0.005 mg/ml) in a total volume of 0.1 ml. The mixture was incubated at 25 °C and in regular time intervals an aliquot was removed and tested for released phosphate. The phosphate assay is described above in further detail.

The results of the phosphate assay are shown in Table 3. The amount of release phosphate was detected.

The enzymes correspond to the following sequences SEQ ID NO: 31, SEQ ID NO: 33, SEQ ID NO: 35, SEQ ID NO: 37, SEQ ID NO: 49, SEQ ID NO: 51.

**Table 3: Comparison of Escherichia coli phosphatase Ffp and phosphatases from different microorganisms for different substrates occurring in the 4-HMF production process**

| Phosphate [mM] | | | | | | | |
|---|---|---|---|---|---|---|---|
| | EcFfp | EIFfp | BoFfp | WcFfp | EcFfpS178N EcFfpN68E | | blank |
| F6P | 0.17 | 0.14 | 0.08 | 0.13 | 0.09 | 0.12 | 0,07 |
| G6P | 1.03 | 0.11 | 0,03 | 0.05 | 0.03 | 0.05 | 0,01 |
| G1P | 0.02 | 0.07 | 0,03 | 0.05 | 0.02 | 0.01 | 0,01 |
| Gly3P | 0.03 | 0.03 | 0.02 | 0.03 | 0.02 | 0.04 | 0.01 |
| 4-HMF-P | 0.29 | 0.02 | 0.24 | 0.12 | 0.34 | 0.25 | 0.02 |

Additionally, it was possible to identify phosphatase enzymes form different microorganisms that are active against 4-HMF-P and show now or only low activity towards the phosphorylated intermediates in the process.

### Conversion of fructose-6-phosphate into 4-(hydroxymethyl)-2-furancarboxaldehyde phosphate testing several 4-(hydroxymethyl)-2-furancarboxaldehyde phosphate synthases

For monitoring the different activities of the different 4-(hydroxymethyl)-2-furancarboxaldehyde phosphate synthases and the variants for the generation of 4-(hydroxymethyl)-2-furancarboxaldehyde phosphate the direct detection of the product was applied.
*Mj*MfnB - *Methanocaldococcus jannaschii* 4-(hydroxymethyl)-2-Furancarboxaldehyde phosphate synthase (SEQ ID No. 15)
*Mm*MfnB - *Methanococcus maripaludis* 4-(hydroxymethyl)-2-Furancarboxaldehyde phosphate synthase (SEQ ID No. 17)
*Mo*MfnB - *Methanothermococcus okinawensis* 4-(hydroxymethyl)-2-Furancarboxaldehyde phosphate synthase (SEQ ID No. 19)
*Mp*MfnB - *Methanobacterium paludis* 4-(hydroxymethyl)-2-Furancarboxaldehyde phosphate synthase (SEQ ID No. 21)
*Mw*MfnB - *Methanobrevibacter wolinii* 4-(hydroxymethyl)-2-Furancarboxaldehyde phosphate synthase (SEQ ID No. 23)
*Mm*eMfnB - *Methylomonas methanica* 4-(hydroxymethyl)-2-Furancarboxaldehyde phosphate synthase (SEQ ID No. 25)
*Mm*aMfnB - *Methanosarcina mazei* 4-(hydroxymethyl)-2-Furancarboxaldehyde phosphate synthase (SEQ ID No. 27)
*Mt*MfnB - *Methanothermococcus thermolithotrophicus* 4-(hydroxymethyl)-2-Furancarboxaldehyde phosphate synthase (SEQ ID No. 29)

The reaction mixture contained 50 mM TRIS-HCl buffer pH 7.0, 2.5 mM MgCl₂, 10 mM F6P, fructose-6-phosphate aldolase from *Escherichia coli* (2 mg/ml) and one 4-(hydroxymethyl)-2-furancarboxaldehyde phosphate synthase (0.5 mg/ml) in a total volume of 0.2 ml. The mixture was incubated at 22 or 45 °C and measured continuously for formation of 4-(hydroxymethyl)-2-furancarboxaldehyde phosphate at 280 nm in a spectrophotometer (Multiskan GO, Thermo Fisher) using 96 well UV transparent bottom F-bottom plates (655801 Greiner Bio-One).

Alternatively, the reaction mixture contained 50 mM TRIS-HCl buffer pH 7.0, 2.5 mM MgCl₂, 10 mM glyceraldehyde-3-phosphate and 4-(hydroxymethyl)-2-furancarboxaldehyde phosphate synthase (0.5 mg/ml) in a total volume of 0.2 ml. The mixture was incubated at 22 or 45 °C and measured continuously for formation of 4-(hydroxymethyl)-2-furancarboxaldehyde phosphate at 280 nm in a spectrophotometer (Multiskan GO, Thermo Fisher) using 96 well UV transparent bottom F-bottom plates (655801 Greiner Bio-One).

Formation of 4-(hydroxymethyl)-2-furancarboxaldehyde phosphate and 4-HMF were also checked using thin layer chromatography. 4-(hydroxymethyl)-2-furancarboxaldehyde phosphate and 4-HMF could be detected with UV light and also with thymol spray reagent solution (0.5 g, 95 ml ethanol, 5 ml H₂SO₄) using the running buffer (15 ml isopropanol, 24.5 ml ethyl acetate, 0.5 ml acetic acid, 10 ml H₂O).

The results of the 4-(hydroxymethyl)-2-furancarboxaldehyde phosphate formation are shown in Table 4. The amount of 4-HMF-P was detected at 280 nm because of the aromatic ring.

**Table 4: Comparison of different 4-(hydroxymethyl)-2-furancarboxaldehyde phosphate synthases from different microorganisms**

| **Time (min)** | **Mj MfnB** | **Mm MfnB** | **Mo MfnB** | **Mp MfnB** | **Mw MfnB** | **Mme MfnB** | **Mma MfnB** | **Mt MfnB** |
|---|---|---|---|---|---|---|---|---|
| **0** | 0.36 | 0.19 | 0.09 | 0.12 | 0.17 | 0.10 | 0.19 | 0.13 |
| **5** | 0.45 | 0.34 | 0.15 | 0.19 | 0.26 | 0.15 | 0.23 | 0.22 |
| **10** | 0.56 | 0.42 | 0.16 | 0.20 | 0.30 | 0.19 | 0.25 | 0.29 |
| **15** | 0.59 | 0.45 | 0.20 | 0.22 | 0.35 | 0.20 | 0.26 | 0.32 |
| **20** | 0.61 | 0.47 | 0.23 | 0.25 | 0.39 | 0.21 | 0.28 | 0.36 |
| **25** | 0.61 | 0.49 | 0.26 | 0.26 | 0.42 | 0.22 | 0.31 | 0.39 |
| **30** | 0.62 | 0.51 | 0.28 | 0.29 | 0.54 | 0.23 | 0.34 | 0.42 |
| **Difference 30-0** | 0.28 | 0.32 | 0.19 | 0.17 | 0.37 | 0.13 | 0.15 | 0.29 |

Under described conditions MwMfnB showed the best conversion, followed by MmMfnB, MtMfnB and MjMfnB. All tested 4-(hydroxymethyl)-2-furancarboxaldehyde phosphate synthases showed activity for the formation of 4-(hydroxymethyl)-2-furancarboxaldehyde phosphate and can be used in a process for 4-(hydroxymethyl)-2-furancarboxaldehyde production.

### Conversion of starch into 4-HMF

Conversion experiments were performed at a 15 ml scale using 50 ml falcon tubes with 1 gram of native starch. Native starch was pretreated with a volume of 6 ml water and buffer and heated up to 95 °C for 30 min to allow an almost complete swelling. Afterwards, the swollen starch was cooled down to 25 °C and the enzymes were added. To 1 gram of native starch a volume of 13.5 ml of buffer, salts and enzymes was added. The solution contained the combination of enzymes as shown in Table 5. The final mixture contained 50 mM KPi pH 7.0, 5 mM MgCl₂, 0.1 mM PLP. Starting with 0 hours' incubation on a regular basis samples were taken and filtered using a 10 kDa spin filter (VWR 82031-350). If necessary samples were diluted in for further analytics. Thin layer chromatography was used to determine the conversion as described by Wang et al. (Wang, Y., Jones, M. K., Xu, H., Ray, W. K., & White, R. H. (2015). Mechanism of the enzymatic synthesis of 4-(Hydroxymethyl)-2-furancarboxaldehyde-phosphate (4-HFC-P) from Glyceraldehyde-3-phosphate catalyzed by 4-HFC-P synthase. Biochemistry, 54(19), 2997-3008.)

**Table 5: Enzymes used for biotransformation of starch to 4-HMF**

| Enzyme | Protein concentration (mg/ml) |
|---|---|
| BfPulA | 0.01 |
| MrAgt | 0.01 |
| AaAgp or MrAgp | 0.25 |
| MrPgm | 0.2 |
| MrPgi | 0.05 |
| EcFsaA | 4.0 |
| MwMfnB | 0.75 |
| EcFfpS178N | 0.005 |

The results of the 4-(hydroxymethyl)-2-furancarboxaldehyde formation are shown in Table 6. The amount of 4-HMF was detected at 280 nm because of the aromatic ring.

**Table 6:**

| **Time [h]** | **4-HMF [mM]** |
|---|---|
| 0 | 0 |
| 2 | 1.9 |
| 4 | 3.6 |
| 6 | 5.4 |
| 8 | 7.1 |
| 24 | 12.2 |

### 4. Conversion of cellulose to 4-HMF

The cell-free bioproduction of 4-HMF was performed in 15 ml scale I scale using 50 ml falcon tubes with 7 grams of regenerated amorphous cellulose. Microcrystalline cellulose was pretreated like described by Zhang et al. (Zhang, Y. H. P., Cui, J., Lynd, L. R., & Kuang, L. R. (2006). A transition from cellulose swelling to cellulose dissolution by o-phosphoric acid: evidence from enzymatic hydrolysis and supramolecular structure. Biomacromolecules, 7(2), 644-648.). The solution contained the combination of enzymes as shown in Table 7. The final mixture contained 50 mM KPi pH 7.0, 5 mM MgCl₂. Starting with 0 hours' incubation on a regular basis samples were taken and filtered using a 10 kDa spin filter (VWR 82031-350). If necessary samples were diluted in for further analytics. Thin layer chromatography was used to determine the conversion as described by Wang et al. (Wang, Y., Jones, M. K., Xu, H., Ray, W. K., & White, R. H. (2015). Mechanism of the enzymatic synthesis of 4-(Hydroxymethyl)-2-furancarboxaldehyde-phosphate (4-HFC-P) from Glyceraldehyde-3-phosphate catalyzed by 4-HFC-P synthase. Biochemistry, 54(19), 2997-3008.)

**Table 7: Enzymes used for biotransformation of cellulose to 4-HMF**

| Enzyme | Protein concentration (mg/ml) |
|---|---|
| CtCdeP | 0.25 |
| CtCelP | 0.25 |
| MrPgm | 0.2 |
| MrPgi | 0.05 |
| EcFsaA | 4.0 |
| MwMfnB | 0.75 |
| EcFfpS178N | 0.005 |

The results of the 4-(hydroxymethyl)-2-furancarboxaldehyde formation are shown in Table 8.

**Table 8:**

| **Time [h]** | **4-HMF [mM]** |
|---|---|
| 0 | 0 |
| 2 | 1.1 |
| 4 | 2.7 |
| 6 | 3.9 |
| 8 | 6.3 |
| 24 | 8.5 |

### 6. Conversion of sucrose to 4-HMF

The cell-free bioproduction of 4-HMF was performed in 1000 µl scale in Eppendorf tube. The solution contained the combination of enzymes as shown in Table 9. Potassium phosphate buffer 50 mM pH 7.0, MgCl₂ 5 mM, sucrose 700 mM at 25°C. The reaction was carried out in triplicates. The formation of 4-HMF was followed over time by withdrawing 100 µl of aliquot at certain time intervals. Starting with 0 hours' incubation on a regular basis samples were taken and filtered using a 10 kDa spin filter (VWR 82031-350).

**Table 9: Enzymes used for biotransformation of sucrose to 4-HMF**

| Enzyme | Protein concentration (mg/ml) |
|---|---|
| BaScp | 0.05 |
| MrPgm | 0.2 |
| MrPgi | 0.05 |
| EcFsaA | 4.0 |
| MwMfnB | 0.75 |
| EcFfpS178N | 0.005 |

The results of the 4-(hydroxymethyl)-2-furancarboxaldehyde formation are shown in Table 10.

**Table 10: Conversion of sucrose to 4-HMF**

| **Time [h]** | **4-HMF (mM)** |
|---|---|
| 0 | 0 |
| 2 | 2 |
| 4 | 5 |
| 6 | 6.5 |
| 8 | 7.2 |
| 24 | 13.5 |

### 7. Conversion of glucose-1-phosphate to 4-HMF with simultaneous conversion of dihydroxyacetone into fructose

The cell-free bioproduction of 4-HMF was performed in 1000 µl scale in Eppendorf tube. The solution contained the combination of enzymes as shown in Table 11. Potassium phosphate buffer 50 mM pH 7.0, MgCl₂ 5 mM, glucose-1-phoshate 100 mM at 25°C. The reaction was carried out in triplicates. The formation of 4-HMF was followed over time by withdrawing 100 µl of aliquot at certain time intervals. Starting with 0 hours' incubation on a regular basis samples were taken and filtered using a 10 kDa spin filter (VWR 82031-350).

Additionally, a phosphate assay was performed to monitor the release of phosphate during the conversion of glucose-1-phosphate. The phosphate assay consists of three solutions, solution A (12 % (w/v) L-ascorbic acid in 1N HCl solution), solution B (2 % (w/v) Na₂MoO₄ x 2H₂O in ddH₂O), solution F (2 % (w/v) citric acid and 2 % (w/v) acetic acid in ddH₂O). Solution D is a 2:1 mixture of solution A and solution B and prepared freshly before the measurement. 75 µl of solution D is pipetted into the needed wells of a flat-bottom microtiter plate. In the next step 25 µl of the sample or a standard with defined concentration is added and incubated for 5 min at room temperature. In the last step the reaction is stopped by addition of 75 µl of solution F and an additional incubation step of 15 min at room temperature. The samples are measured at 655 nm in a spectrophotometer (Multiskan GO, Thermo Fisher).

**Table 11: Enzymes used for biotransformation of glucose-1-phosphate to 4-HMF**

| Enzyme | Protein concentration (mg/ml) |
|---|---|
| MrPgm | 0.2 |
| MrPgi | 0.05 |
| EcFsaA | 4.0 |
| MwMfnB | 0.75 |
| BoFfp | 0.01 |
| EcFsaAA129S | 4.0 |
| PwHyi | 1.2 |

The results of the 4-(hydroxymethyl)-2-furancarboxaldehyde formation are shown in Table 12.

**Table 12: Conversion of glucose-1-phosphate to 4-HMF**

| **Time [h]** | **4-HMF (mM)** | **Fructose (mM)** |
|---|---|---|
| 0 | 0 | 0 |
| 2 | 3.1 | 1.7 |
| 4 | 6.2 | 4.2 |
| 6 | 7.8 | 5.6 |
| 8 | 11.2 | 7.1 |
| 24 | 19.5 | 15.9 |

### 8. Conversion of sucrose to 4-HMF with simultaneous conversion of dihydroxyacetone into fructose

The cell-free bioproduction of 4-HMF was performed in 1000 µl scale in Eppendorf tube. The solution contained the combination of enzymes as shown in Table 13. Potassium phosphate buffer 50 mM pH 7.0, MgCl₂ 5 mM, sucrose 700 mM at 25°C. The reaction was carried out in triplicates. The formation of 4-HMF was followed over time by withdrawing 100 µl of aliquot at certain time intervals. Starting with 0 hours' incubation on a regular basis samples were taken and filtered using a 10 kDa spin filter (VWR 82031-350). Thin layer chromatography was used to determine the conversion as described by Wang et al. (Wang, Y., Jones, M. K., Xu, H., Ray, W. K., & White, R. H. (2015). Mechanism of the enzymatic synthesis of 4-(Hydroxymethyl)-2-furancarboxaldehyde-phosphate (4-HFC-P) from Glyceraldehyde-3-phosphate catalyzed by 4-HFC-P synthase. Biochemistry, 54(19), 2997-3008.)

**Table 13: Enzymes used for biotransformation of sucrose to 4-HMF with simultaneous conversion of dihydroxyacetone into fructose**

| Enzyme | Protein concentration (mg/ml) |
|---|---|
| BaScp | 0.05 |
| MrPgm | 0.2 |
| MrPgi | 0.05 |
| EcFsaA | 4.0 |
| MwMfnB | 0.75 |
| EcFfpS178N | 0.005 |
| EcFsaAA129S | 4.0 |
| PwHyi | 1.2 |

The results of the 4-(hydroxymethyl)-2-furancarboxaldehyde formation are shown in Table 14.

**Table 14: Conversion of sucrose to 4-HMF**

| **Time [h]** | **4-HMF (mM)** |
|---|---|
| 0 | 0 |
| 2 | 4.2 |
| 4 | 7.3 |
| 6 | 9.1 |
| 8 | 13.6 |
| 24 | 22.8 |

### 9. Conversion of sucrose to 4-HMF with simultaneous conversion of dihydroxyacetone into fructose

The cell-free bioproduction of 4-HMF was performed in 1000 µl scale in Eppendorf tube. The solution contained the combination of enzymes as shown in Table 15 Potassium phosphate buffer 50 mM pH 7.0, MgCl₂ 5 mM, sucrose 700 mM at 25°C. The reaction was carried out in triplicates. The formation of 4-HMF was followed over time by withdrawing 100 µl of aliquot at certain time intervals. Starting with 0 hours' incubation on a regular basis samples were taken and filtered using a 10 kDa spin filter (VWR 82031-350). Thin layer chromatography was used to determine the conversion as described by Wang et al. (Wang, Y., Jones, M. K., Xu, H., Ray, W. K., & White, R. H. (2015). Mechanism of the enzymatic synthesis of 4-(Hydroxymethyl)-2-furancarboxaldehyde-phosphate (4-HFC-P) from Glyceraldehyde-3-phosphate catalyzed by 4-HFC-P synthase. Biochemistry, 54(19), 2997-3008.)

**Table 15: Enzymes used for biotransformation of sucrose to 4-HMF with simultaneous conversion of dihydroxyacetone into fructose**

| Enzyme | Protein concentration (mg/ml) |
|---|---|
| BaScp | 0.05 |
| MrPgm | 0.2 |
| MrPgi | 0.05 |
| EcFsaA | 4.0 |
| MwMfnB | 0.75 |
| EcFfpS178N | 0.005 |
| EcFsaAA129S | 4.0 |
| EcOtnI | 1.2 |

The results of the 4-(hydroxymethyl)-2-furancarboxaldehyde formation are shown in Table 16.

**Table 16: Conversion of sucrose to 4-HMF with simultaneous conversion of dihydroxyacetone into fructose**

| **Time [h]** | **4-HMF (mM)** |
|---|---|
| 0 | 0 |
| 2 | 3.3 |
| 4 | 5.9 |
| 6 | 7.8 |
| 8 | 11.9 |
| 24 | 17.4 |

## Claims

1. A process for producing 4-hydroxymethylfurfural (HMF) comprising the steps of:
- converting 4-hydroxymethylfurfural-phosphate to 4-hydroxmethylfurfural using a phosphatase.

2. Process according to claim 1,
wherein the phosphatase is obtained from an organism, preferably a microorganism, and more preferably from a microorganism selected from the group consisting of *Escherichia coli* and/or *Blautia obeum;* and/or
wherein the phosphatase has an activity towards other compounds present in the process being less than or equal to 10% compared to the activity towards 4-hydroxymethylfurfural-phosphate; and/or
wherein the conversion of 4-hydroxymethylfurfural-phosphate to 4-hydroxmethylfurfural is an irreversible reaction.

3. Process according to any of the preceding claims,
wherein the phosphate created in the conversion from 4-hydroxymethylfurfural-phosphate to 4-hydroxmethylfurfural is recycled; and/or
wherein in the conversion from 4-hydroxymethylfurfural-phosphate to 4-hydroxmethylfurfural no co-factor, preferably no NAD⁺/NADH, NADP⁺/NADPH, FAD, FMN or ATP, is needed.

4. Process according to any of the preceding claims,
wherein 4-hydroxymethylfurfural-phosphate is prepared from glyceraldehyde-3-phosphate using at least one enzyme; or
wherein 4-hydroxymethylfurfural-phosphate is prepared from glyceraldehyde-3-phosphate using at least one enzyme and wherein the phosphate created in the conversion is recycled in the process.

5. Process according to claim 4,
wherein the at least one enzyme is a synthase; or
wherein the at least one enzyme is a synthase and wherein the synthase is obtained from an organism, preferably a microorganism and more preferably from a microorganism selected from the group consisting of *Methanocaldococcus jannaschii, Methanococcus maripaludis, Methanothermococcus okinawensis, Methanobacterium paludism, Methanobrevibacter wolinii, Methylomonas methanica, Methanosarcina mazei, and Methanothermococcus thermolithotrophicus.*

6. Process according to claims 4 or 5,
wherein the glyceraldehyde-3-phosphate is prepared from fructose-6-phosphate using at least one enzyme, wherein this reaction also yields dihydroxyacetone in a non-phosphorylated form; or
wherein the glyceraldehyde-3-phosphate is prepared from fructose-6-phosphate using at least one enzyme, wherein this reaction also yields dihydroxyacetone in a non-phosphorylated form, and wherein the dihydroxyacetone is isomerized to glyceraldehyde using an isomerase, wherein preferably the isomerase is an isomerase belonging to the EC5.3.1 class, and more preferably the isomerase is an isomerase belonging to the EC5.3.1.22 class or EC5.3.1.35 class.

7. Process according to claim 6,
wherein the dihydroxyacetone is further converted into fructose, preferably the mixture of dihydroxyacetone and glyceraldehyde is converted into fructose.

8. Process according to any of the claims 5 to 7,
wherein the synthase comprises an amino acid sequence that is at least 80 %, preferably at least 90 %, more preferably at least 98 %, and even more preferably at least 99% identical with the sequence according to SEQ ID NO: 23; or
wherein the synthase has an amino acid sequence according to SEQ ID NO: 15, SEQ ID NO: 17, SEQ ID NO: 19, SEQ ID NO: 21, SEQ ID NO: 25, SEQ ID NO: 27, or SEQ ID NO: 29.

9. Process according to any of the preceding claims,
wherein the process is cell-free; and/or
wherein the process is a one-pot reaction; and/or
wherein the process is part of a multi-enzyme reaction cascade.

10. Process according to any of the preceding claims,
wherein the phosphatase comprises an amino acid sequence that is at least 95 %, preferably at least 98 %, more preferably at least 98.5 % and even more preferably at least 99 % identical with the sequence according to SEQ ID NO: 51, preferably the phosphatase is derived from *E. coli;* or
wherein the phosphatase comprises an amino acid sequence that is at least 80 %, preferably at least 90 %, and more preferably at least 98 % and even more preferably at least 99 % identical with the sequence according to SEQ ID NO: 35, preferably the phosphatase is derived from a *Blautia obeum.*

11. A process for producing 4-HMF from a feedstock comprising a carbon source, comprising the steps of
- converting 4-hydroxymethylfurfural-phosphate to 4-hydroxmethylfurfural using a phosphatase;
wherein the carbon source is preferably selected from the group consisting of starch, dextrines, cellulose, sucrose and/or combinations thereof.

12. Process for producing 4-HMF from a feedstock comprising a carbon source according to claim 11,
wherein the process is a multi-enzyme reaction cascade; and/or
wherein the process comprises at least five enzymes; and/or
wherein the phosphate created in the production of 4-hydroxmethylfurfural is recycled in the production process; and/or
wherein in the process no co-factor, preferably no NAD⁺/NADH, NADP⁺/NADPH, FAD, FMN or ATP, is needed; and/or
wherein the phosphatase has an activity towards other compounds present in the process being less than or equal to 10% compared to the activity towards 4-hydroxymethylfurfural-phosphate, preferably the phosphatase has an activity towards glucose-1-phosphate, glucose-6-phosphate, fructose-6-phosphate and glyceraldehye-3-phosphate being less than or equal to 10% compared to the activity towards 4-hydroxymethylfurfural-phosphate.

13. Phosphatase for converting 4-hydroxymethylfurfural-phosphate to 4-hydroxmethylfurfural comprising an amino acid sequence that is at least 95 %, preferably at least 98 %, more preferably at least 98.5 % and even more preferably at least 99 % identical with the sequence according to SEQ ID NO: 51, preferably the phosphatase is derived from *E. coli;* or
phosphatase for converting 4-hydroxymethylfurfural-phosphate to 4-hydroxmethylfurfural comprising an amino acid sequence that is at least 80 %, preferably at least 90 %, and more preferably at least 98 % and even more preferably at least 99 % identical with the sequence according to SEQ ID NO: 35, preferably the phosphatase is derived from a *Blautia obeum.*

14. Phosphatase according to claim 13 having an amino acid sequence according to SEQ ID NO: 35, SEQ ID NO: 37, SEQ ID NO: 47, SEQ ID NO: 49, SEQ ID NO: 51, or SEQ ID NO: 53.

15. Use of a phosphatase according to claims 13 or 14 in the production of 4-hydroxmethylfurfural.
